# EUROPEAN PATENT APPLICATION

(11) **EP 4 756 833 A1**
(43) Date of publication of application: **10.06.2026**
(21) Application number: 23947235.0
(22) Date of filing: 01.09.2023
(51) Int. Cl.: G16H 40/67, G01N 33/66, H04W 76/14, H04W 4/80, A61B 5/145, H04L 67/12

(54) **DIABETES MANAGEMENT SYSTEM USED IN HOSPITAL**

(30) Priority: 31.07.2023 WO PCT/CN2023/110187
(71) Applicant: Medtrum Technologies Inc., Shanghai 201203 (CN)
(72) Inventor: YANG, Cuijun, Shanghai 201203 (CN)
(74) Representative: Vogelbruch, Keang
(86) International application number: PCT/CN2023/116457
(87) International publication number: WO 2025/025293

(57) **Abstract**

The invention discloses a diabetes management system for use in a hospital, including: at least a wearable medical device, a plurality of receivers, a central server and a healthcare provider terminal, the receivers receive the data information transmitted by the medical device and upload it to the central server, and the healthcare provider communicates with the central server and displays or reviews the medical data information through the terminal, as any one of the multiple receivers can receive the data information transmitted by the medical device and upload it to the central server, as a result, the patient's range of motion in the hospital is no longer restricted, which improves the patient's experience and keeps the patient in a comfortable mood, while the healthcare provider is able to know the patient's blood glucose level and/or insulin infusion state in real time through the healthcare provider terminal and deal with the abnormalities in a timely manner, so that the patient's blood glucose level can be maintained at a stable level.

## Description

### TECHNICAL FIELD

The present invention mainly relates to the field of diabetes monitoring and management, and in particular, to a glucose management system for use in a hospital.

### BACKGROUND

The pancreas of healthy people can automatically secrete the required insulin/glucagon according to the glucose level in the human blood, thereby maintaining a reasonable range of blood glucose fluctuations. However, for diabetic patients, the function of their pancreas has been severely compromised, and the pancreas cannot secrete the required dosage of insulin. Therefore, diabetes mellitus is defined as a metabolic disease caused by abnormal pancreatic function, and it is also classified as one of the top three chronic conditions by the WHO. The present medical advancement has not been able to find a cure for diabetes mellitus. Yet, the best the technology could do is control the onset symptoms and complications by stabilizing the blood glucose level for diabetes patients.

Diabetic patients on an insulin pump need to check their blood glucose before infusing insulin into their bodies. At present, most detection methods of continuously monitoring blood glucose are based on in vivo glucose monitoring devices, which use disposable transdermal sensors inserted into the skin to measure glucose concentrations in interstitial fluids and send the blood glucose data through a transmitter to the remote device in real-time for the user to review. This monitoring method is called Continuous Glucose Monitoring (CGM).

Diabetic patients with mild symptoms can complete self-monitoring and management of blood glucose through continuous glucose monitoring, while diabetic patients with more severe symptoms need to be treated by the doctor in the hospital, such as adopting targeted treatment plans to restore the blood glucose level of diabetic patients to a relatively stable state, and at the same time, providing personalized medication delivery, diet and exercise plans to facilitate the patients to achieve self-monitoring and management of blood glucose even after they have been discharged from the hospital.

The diabetes management system currently used in hospitals also consists of a disposable transdermal sensor worn on the patients to continuously monitor blood glucose data, connects and transmits the data to a device that receives the data (hereinafter referred to as the receiver) through a transmitter, and then sends the blood glucose data from the receiver to the healthcare provider's terminal equipment and displays it, so the healthcare provider can review the patient's blood glucose data. After providing personalized insulin infusion plan for different patients, the healthcare provider will infuse insulin into the patient's body through the insulin pump, and at the same time, the insulin pump will send the infused insulin information to the receiver, and further send it to the healthcare provider terminal and display it, so that the healthcare provider can review the patient's insulin infusion information.

In some diabetes management systems, the data from multiple transmitters are transmitted to the same receiver, and in some diabetes management systems, the data from one transmitter are transmitted to a corresponding receiver. Currently, the in vivo blood glucose monitoring devices typically have a lifespan of 7-14 days, insulin pump typically have a lifespan of 3 days, and in order to save power and extend the lifespan of the devices, the medical devices typically communicate with the receiver via Bluetooth. In general, the effective communication distance range of Bluetooth is 0-20m, when a medical device corresponds to a receiver, if the patient wears the medical device and leaves the ward, the distance range between the medical device and the receiver is likely to exceed the effective communication distance of the medical device, resulting in the receiver not being able to receive real-time blood glucose data and/or insulin infusion information and upload it to and display on the healthcare provider terminal. As a result, the motion range of a diabetic patient in the hospital is usually restricted to a specific ward, which not only creates a strong sense of being confined to the diabetic patient', but may also interfere with the patient's other medical detection or surgeries. When all information from multiple devices is transmitted to the same receiver, not only is there the same problem of communication distance limitation, but at the same time, if the relationship between the multiple medical devices and the patient information are not matched, the blood glucose data and/or insulin infusion information received by the receiver cannot be identified and categorized in accordance with the patients, which results in the problem of chaotic matching of the blood glucose data and/or insulin infusion information with the patients, further affecting the correct treatment of the patient by the doctor and endangering the patient's life safety.

Therefore, in the prior art, there is an urgent need for a diabetes management system for use in a hospital which not only would not restrict a patient's range of motion and cause a feeling of constriction to the patient, but also could correctly matching the patients with the blood glucose data and/or insulin infusion information.

### BRIEF SUMMARY OF THE INVENTION

The embodiment of the present invention discloses a diabetes management system for use in a hospital. The system comprises at least a wearable medical device, a plurality of receivers, a central server and a healthcare provider terminal, the receivers receive the data information transmitted by the medical device and upload it to the central server, and the healthcare provider communicates with the central server and displays or reviews the medical data information through the terminal, as any one of the multiple receivers can receive the data information transmitted by the medical device and upload it to the central server, as a result, the patient's range of motion in the hospital is no longer restricted, which improves the patient's experience and keeps the patient in a comfortable mood, while the healthcare provider is able to know the patient's blood glucose level and/or insulin infusion state in real time through the device used by the healthcare provider and deal with the abnormalities in a timely manner, so that the patient's blood glucose level can be maintained at a stable level.

The invention discloses a diabetes glucose management system for use in a hospital, comprising: at least one wearable medical device worn by at least a patient, the wearable medical device comprises at least a memory, a processor and a communication interface, and the communication interface is configured to transmit and receive information; a plurality of receivers, any of which is configured to receive and relay information transmitted by the wearable medical device; a central server, comprising a memory and a communication interface configured to communicate with an external device and store information received from the external device; and a healthcare provider terminal, configured to communicate with the central server and to display or review medical data information.

According to one aspect of the present invention, the at least one wearable medical device comprises a CGM and/or an insulin pump.

According to one aspect of the present invention, when a position of the at least one wearable medical device changes with the patient in the hospital, the information transmitted by the wearable medical device is received by the receiver located at an optimal communication distance.

According to one aspect of the present invention, each wearable medical device is provided with a unique identifier and the information transmitted by the wearable medical device at least comprises the identifier information.

According to one aspect of the present invention, the healthcare provider binds and uploads the patient's personal information and the identifier information to the central server via the healthcare provider terminal.

According to one aspect of the present invention, the identifier includes at least one of a device identifier, a hardware identifier, a generic unique identifier, a serial number, a communication protocol-based identifier, a manufacturer's identifier.

According to one aspect of the present invention, the identifier is provided on an outer packaging or housing of the wearable medical device.

According to one aspect of the present invention, the wearable medical device at least comprises a reusable part, and the identifier is provided on the outer packaging or housing of the reusable part.

According to one aspect of the present invention, the identifier is set in the form of QR codes, bar codes, or NFC tags
According to one aspect of the present invention, the healthcare provider terminal comprises one or more processors that enable multiple individuals with different access level rights to modify the setup parameters of the healthcare provider terminal.

According to one aspect of the present invention, the healthcare provider terminal is a mobile device and the CGM and/or the insulin pump is controlled by the same APP in the mobile device.

According to one aspect of the present invention, the healthcare provider terminal further comprises a displayer, at least used for displaying one of the patient's real-time glucose data, historical glucose trend graph or insulin infusion information.

According to one aspect of the present invention, the healthcare provider terminal is a HIS system, configured to review, print or delete blood glucose and/or insulin infusion reports.

According to one aspect of the present invention, the healthcare provider terminal is a monitoring screen, for displaying blood glucose and/or insulin infusion information for all patients being monitored.

According to one aspect of the present invention, the healthcare provider terminal is a monitoring screen, configured to display blood glucose and/or insulin infusion information for all patients being monitored.

Compared with the prior art, the technical solution of the present invention has the following advantages:
In the diabetes management system for use in a hospital disclosed in the present invention, it comprises at least a wearable medical device, a plurality of receivers, a central server and a healthcare provider terminal, the receivers receive the data information transmitted by the medical device and upload it to the central server, and the healthcare provider communicates with the central server and displays or reviews the medical data information through the device, as any one of the multiple receivers can receive the data information transmitted by the medical device and upload it to the central server, as a result, the patient's range of motion in the hospital is no longer restricted, which improves the patient's experience and keeps the patient in a comfortable mood, while the healthcare provider is able to know the patient's blood glucose level and/or insulin infusion state in real time through the device used by the healthcare provider and deal with the abnormalities in a timely manner, so that the patient's blood glucose level can be maintained at a stable level.

Furthermore, the data information transmitted by the medical device worn by the patient can be received at any time by the receiver located within the optimal communication distance, on the one hand, it can save the power consumption of the medical device, on the other hand, it can prevent each receiver from receiving the data and uploading it to the central server, which will result in the duplication of the blood glucose data information and/or insulin infusion information, and occupies the storage space of the central server.

Furthermore, each wearable medical device is set with a unique identifier, the medical device sends blood glucose data information and/or the insulin infusion information and the identifier information, the healthcare provider binds the patient's personal information and identifier information through the device used by them and uploads it to the central server, which stores the corresponding blood glucose and/or the insulin infusion information and identifier information, and the corresponding patient's personal information and identifier information. Therefore, by the identifier information, one can match the patient's personal information and the blood glucose and/or the insulin infusion information, avoiding mismatching the blood glucose data and/or the insulin infusion with the patient, thus affecting the doctor's correct treatment of the patient and jeopardizing the patient's life safety.

Furthermore, the healthcare provider terminal comprises one or more processors that enable multiple individuals with different access level rights to modify the setup parameters of the healthcare provider terminal, which can prevent misuse by someone who do not have the appropriate level of authority, thus to ensure the safety of patient diabetes treatment.

Furthermore, the healthcare providers can use a single APP in the same mobile device to control the CGM and/or insulin pump at the same time and review the patient's blood glucose and/or insulin infusion information, which is convenient for healthcare providers to use and improves the healthcare providers ' experience.

Furthermore, the diabetes management system for use in a hospital includes a personal diabetes management device, the healthcare provider can limit the patient's operation on the personal diabetes management device through a locking mode, on the one hand, facilitating the patient to know his/her blood glucose information and/or insulin infusion information in the first instance, learn and become proficient in the use of the personal diabetes management device, and continue the use of the management device by himself/herself to monitor and manage his/her blood glucose level after discharged from the hospital, on the other hand, patient actions on the personal diabetes management device can also be limited to specific time ranges by a locking mode to prevent misuse by the patient.

### BRIEF DESCRIPTION OF THE DRAWINGS

FIG.1 is a topological diagram of the diabetes management system for use in hospitals according to one embodiment of the present invention.
FIG.2 is a schematic diagram of the integrated continuous glucose monitoring device according to an embodiment of the present invention.
FIG.3 is a schematic diagram of the split continuous glucose monitoring device according to an embodiment of the present invention.
FIG.4a is a schematic diagram of the integrated insulin pump according to an embodiment of the present invention.
FIG.4b is a schematic diagram of the split insulin pump according to an embodiment of the present invention.
FIG.5 is a comparison diagram of information stored in the central server according to an embodiment of the present invention.
FIG. 6 is a default home screen of the APP of the mobile phone according to an embodiment of the present invention.

### DETAILED DESCRIPTION

As mentioned above, the diabetes management systems used in hospitals in prior art are those in which data from a plurality of medical devices is transmitted to the same receiver, or in which one medical device sends data to a corresponding receiver. Due to the limitation of the communication distance of the medical devices, the patient's range of motion is likely to be restricted to the ward, which not only creates a strong sense of constriction to the patient, but also may interfere with the patient's other examinations or surgeries.

In order to solve this problem, the present invention provides a diabetes management system for use in a hospital. The system comprises at least a wearable medical device, a plurality of receivers, a central server and a healthcare provider terminal, the receivers receive the data information transmitted by the medical device and upload it to the central server, and the healthcare provider communicates with the central server and displays or reviews the medical data information through the device, as any one of the multiple receivers can receive the data information transmitted by the medical device and upload it to the central server, as a result, the patient's range of motion in the hospital is no longer restricted, which improves the patient's experience and keeps the patient in a comfortable mood, while the healthcare provider is able to know the patient's blood glucose level and/or insulin infusion state in real time through the device used by the healthcare provider and deal with the abnormalities in a timely manner, so that the patient's blood glucose level can be maintained at a stable level.

Various exemplary embodiments of the present invention will now be described in detail with reference to the drawings. The relative arrangement of the components and the steps, numerical expressions and numerical values set forth in the embodiments are not to be construed as limiting the scope of the invention.

In addition, it should be understood that, for ease of description, the dimensions of the various components shown in the figures are not necessarily drawn in the actual scale relationship, for example, the thickness, width, length or distance of certain units may be exaggerated relative to other parts.

The following description of the exemplary embodiments is merely illustrative, and is not intended to be in any way limiting the invention and its application or use. The techniques, methods, and devices that are known to those of ordinary skill in the art may not be discussed in detail, but such techniques, methods, and devices should be considered as part of the specification.

It should be noted that similar reference numerals and letters indicate similar items in the following figures. Therefore, once an item is defined or illustrated in a drawing, it will not be discussed further in the following description of the drawings.

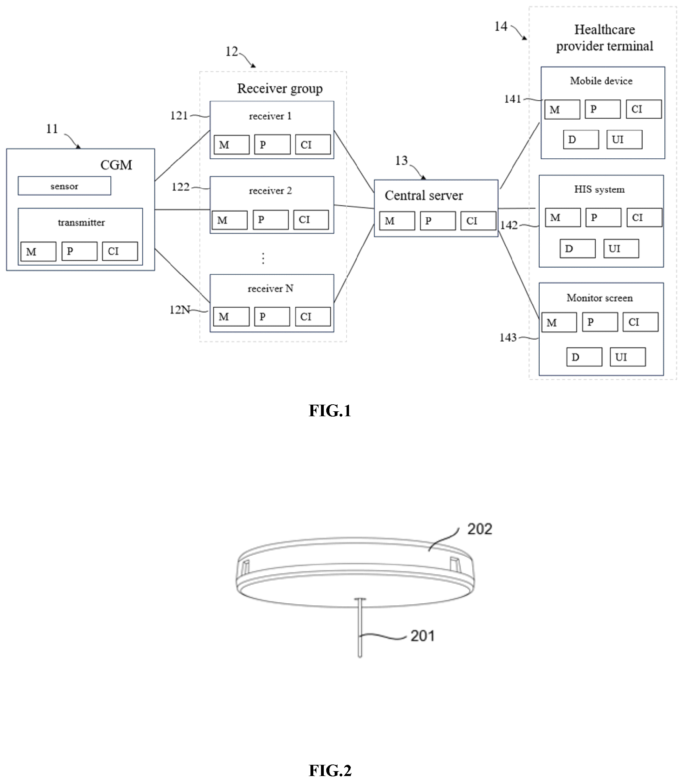

FIG.1 is a topological diagram of the diabetes management system for use in hospitals according to the embodiment of the present invention.

In embodiments of the present invention, a diabetes management system for use in a hospital includes: a wearable medical device 11, a receiver group 12 including a plurality of receivers, a central server 13, a healthcare provider terminal 14, and the like.

The medical device 11 includes a blood glucose monitoring device and/or an insulin pump, the blood glucose monitoring device used by the patient, is used for monitoring of the patient's blood glucose level in real-time, mounted on the patient by means of an auxiliary applicator, comprising implantable sensors, the sensors being connected to a transmitter, the transmitter further comprising a memory (M), a processor (P), a communication interface (CI), and the like, and the transmitter is used for transmitting the blood glucose data information monitored by the blood glucose monitoring device, as well as an identifier of the blood glucose monitoring device 11, and the like; The insulin pump includes the essential infusion mechanism and electronic control mechanism used to infuse insulin, such as reservoir, drive structures, fluid path and infusion needles, power supplies, circuit boards, and so on. The electronic control unit includes a memory (M), a processor (P), a communication interface (CI), etc. The infusion mechanism is used to injects the current insulin dose required into the user's body. The control mechanism is used to receive insulin infusion commands and transmit insulin infusion status and identifier information of insulin device, and the like.

The receiver group 12 including a plurality of receivers, any one of the plurality of receivers can be used to receive and relay information transmitted by the transmitter, can be distributed in various locations in a hospital such as a ward, a doctor's office, a monitoring room, an operating room, a lavatory, and so on, as needed, and used to receive various types of data information from the wearable medical device 11 such as information about the patient's blood glucose data and/or insulin infusion information, identifier of the wearable medical device 11, and so on, and the receivers include a memory (M), a processor (P), a communication interface (CI), and so on, and can also send the various types of data information received from the transmitter to other devices.

The central server 13, data center or cloud, hereinafter collectively referred to as the central server 13, for storing various types of data and information, including a memory (M), a processor (P), a communication interface (CI), etc., and can receive and store various types of data and information from various receivers, and can also communicate with an external device, which can be a receiver or a healthcare provider terminal 14, etc.

The healthcare provider terminal 14, such as an operating system-containing mobile device 141 for interacting with the central server 13, a hospital information system (HIS system) 142 for reviewing or deleting reports, a monitoring screen 143 for displaying blood glucose information for all patients, and so on, may include a memory (M), a processor (P), a communication interface (CI), a displayer (D), a user interface (UI), and so on.

The wearable medical device 11 and the plurality of receivers of the receiver group 12, the receiver group 12 and the central server 13, the central server 13 and the various terminals 14 used by the healthcare providers are communicated wirelessly. Wireless communications may include but are not limited to, for example, radio frequency (RF) communications (e.g., radio frequency identification (RFID), Zigbee communication protocols, WiFi, infrared, wireless Universal Serial Bus (USB), Ultra Wide Band (UWB), Bluetooth^{®} communication protocols, and cellular communications, such as Code Division Multiple Access (CDMA) or the Global System for Mobile Communications (GSM). Preferably, the wearable medical device 11 and the plurality of receivers of the receiver group 12, the receiver group 12 and the central server 13, the central server 13 and the various healthcare provider terminals 14 are communicated by Wifi and/or cellular communications.

It should be noted that in embodiments of the present invention, communication between the wearable medical device 11 and the multiple the healthcare provider terminals 14 may also be via wireless communication, preferably, via Bluetooth.

No matter where the patient is located in the hospital, the data transmitted by the medical device will be received by the receiver that is at the optimal communication distance, avoiding that all the receivers or a plurality of receivers of the receiver group 12 receive the data, on the one hand, it can save the power consumption of the medical device and, on the other hand, it can prevent each receiver from receiving the data and then uploading the data to the centralized server, which results in the duplication of the blood glucose data information and/ or insulin infusion information and occupies the storage space of the central server.

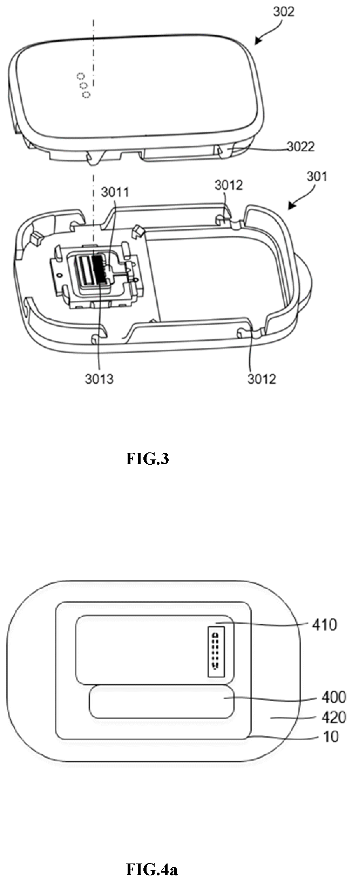

FIG.2 is a schematic diagram of the integrated continuous glucose monitoring device according to an embodiment of the present invention. FIG.3 is a schematic diagram of the split continuous glucose monitoring device according to an embodiment of the present invention.

The blood glucose monitoring device is a wearable continuous glucose monitoring device (hereinafter referred to as CGM), the CGM comprising a sensor and a transmitter, the sensor being used to collect analyte data in the human body and transmit the collected analyte content information, i.e., the sensor is used to collect information on blood glucose data in the human body and transmit the information. The transmitter is connected to the sensor, and it is used to receive the information on the blood glucose data, which is transmitted by the sensor that has been implanted under the skin, and convert the information into a wireless signal for outputting, and at the same time, it can also output other information such as information on an identifier of the CGM. Each CGM has a unique identifier including, but not limited to a device identifier, a hardware identifier, a generic unique identifier, a serial number, an identifier based on communication protocol (e.g., a BLE ID), a manufacturer's identifier, and the like, preferably, identifier information is the serial number of the CGM, which is formed by a plurality of randomly-combined numerical and alphabetic, and can be set on a housing or package of the CGM, or may also be set differently depending on the type of the CGM.

Specifically, in one embodiment of the present invention, the CGM is integrated, i.e., the sensor and the transmitter have been integrated together prior to use, and is a single-use product, which is discarded after use, as shown in FIG. 2. FIG.2 is a schematic diagram of the integrated continuous glucose monitoring device according to an embodiment of the present invention. The integrated continuous glucose monitoring device includes a sensor 201, a housing 202, and a transmitter (not shown in the figure) set inside the housing 202, the sensor 301 is used to monitor the patient's body fluid blood glucose data information, and transmit the said blood glucose data information to the transmitter through an internal circuit, which in turn sends it to a receiver. The identifier may be provided on the outer housing of the CGM or on the outer packaging or within the CGM.

In another embodiment of the present invention, the CGM is split, i.e., the sensor and the transmitter are two different parts prior to use, packaged separately, and only integrated together when in use, as shown in FIG. 3. FIG.3 is a schematic diagram of the split continuous glucose monitoring device according to an embodiment of the present invention. The split continuous glucose monitoring device includes a base 301 and a transmitter 302, the base is provided with a sensor 3011, the transmitter 302 has a separate housing, the housings of the base 301 and the transmitter 302 are respectively provided with snap-in structures 3012 and 3022, the base 301 and the transmitter 302 are snapped into a whole through the snap-in structures when in use, the sensor 3011 is electrically connected to the transmitter 302 through the electrical connection 3013, the sensor 301 is used to monitor the patient's blood glucose data information, the blood glucose data information is transmitted to the transmitter 302 through the electrical connection 3013, and then sent to the receiver by the transmitter 302.

In one embodiment of the present invention, both the sensor and the transmitter of the split continuous glucose monitoring device are single-use products, which are discarded after use, and therefore the identifier may be provided on the housing or the outer packaging of the sensor or the transmitter. In yet another embodiment of the present invention, only the sensor of the split continuous glucose monitoring device is a single-use product and the transmitter is a reusable product, therefore, preferably, in this embodiment, the identifier is provided on the housing or the outer packaging of the transmitter, which reduces the frequency of tying the patient's information to the identifier and improves the patient experience. This will be described in more detail below.

When the identifier is set on the housing or outer packaging of the CGM or transmitter, it is set in a form that includes, but is not limited to, a QR code, a bar code, or an NFC tag.

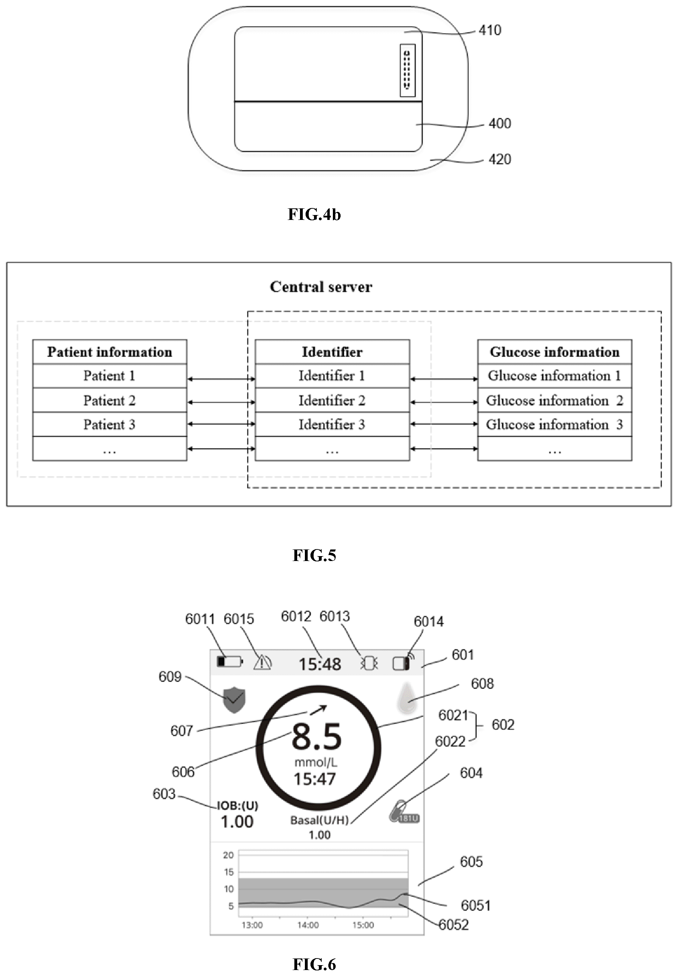

FIG.4a is a schematic diagram of the integrated insulin pump according to an embodiment of the present invention. FIG.4b is a schematic diagram of the split insulin pump according to an embodiment of the present invention.

In this embodiment of the invention, the insulin pump is a patch-type insulin pump, i.e., an insulin pump that does not comprise a long catheter, includes an infusion mechanism and a control mechanism, and being integrally affixed to the surface of the user's skin by an adhesive patch, and the medication is infused directly from the reservoir to the subcutaneous area along the infusion needle.

Each insulin pump has a unique identifier including, but not limited to a device identifier, a hardware identifier, a generic unique identifier, a serial number, an identifier based on communication protocol (e.g., a BLE ID), a manufacturer's identifier, and the like, preferably, the identifier is formed by a plurality of randomly-combined numerical and alphabetic, and can be set on a housing or package of the insulin pump, or may also be set differently depending on the type of the insulin pump.

FIG.4a is a schematic diagram of the integrated insulin pump, i.e., the infusion mechanism 410 and the control mechanism 400 of the insulin pump are provided inside the same housing 10, and both are connected by wires and are affixed to a location on the user's skin by means of an adhesive patch 420, which is disposed of in its entirety after a single use; an identifier may be provided on the outer housing of the insulin pump or on the outer packaging or inside the insulin pump.

FIG.4b is a schematic diagram of the split insulin pump, i.e., the infusion mechanism 410 and the control mechanism 400 of the insulin pump are provided in two different housings, both of which are connected by waterproof plugs or directly snap together and electrically connected as a whole, an identifier may be provided on the outer housing of the infusion mechanism and/or the control mechanism or on the outer packaging or inside the insulin pump.

In one embodiment of the present invention, both the infusion mechanism and the control mechanism of the split insulin pump are single-use products, which are discarded after use, and therefore the identifiers may be provided on the housing or on the outer packaging of the infusion mechanism and/or the control mechanism. In yet another embodiment of the present invention, only the infusion mechanism of the split insulin pump is a single-use product, while the control mechanism is a reusable product, and therefore, preferably, in this embodiment, the identifier is set on the housing of the control mechanism or on the outer packaging, which can reduce the frequency of binding of patient information and identifiers, and improve the patient experience. This is described in more detail below.

When the identifier is set on the housing of the control mechanism or on the outer packaging, it is set in a form that includes, but is not limited to, a QR code, a bar code, or an NFC tag.

When a patient is admitted to the hospital, the healthcare provider creates a new patient account via the mobile device 141, matches the patient's personal information with the identifier of the worn medical device, and uploads the patient's personal information and the corresponding identifier to the central server 13, where the patient's personal information includes name, age, gender, department, bed number, ward, diagnosis, inpatient hospitalization number, cell phone number, and so on. When the medical device worn by the patient needs to be replaced with a new medical device due to reaching its life cycle or failure, etc., the unique identifier information of the new medical device also needs to be updated by pairing with the patient's personal information through the mobile device 141 and further uploaded to the central server 13. The central server 13 stores the patient's personal information and corresponding identifier information. The patient's personal information can be entered manually or by scanning the patient's inpatient bracelet to automatically match the relevant information, and the identifier information can also be entered manually or by scanning the QR code, bar code, or NFC tag on the medical device housing or the outer packaging.

When the CGM is a split CGM and the transmitter is reusable, the CGM identifier is set on the transmitter's housing or packaging, so that when the patient needs to replace the CGM, he or she only needs to replace the sensor, not the transmitter, and the CGM identifier remains unchanged. As a result, there is no need to update the pairing update of the CGM identifier with the patient's personal information via the mobile device 141, and even less need for further uploading to the central server 13, thus reducing the number of operational steps and improving the patient experience.

When the insulin pump is split and the control mechanism is reusable, the identifier of the insulin pump is set on the housing or packaging of the control mechanism, and the patient only needs to change the infusion mechanism when replacing the insulin pump without changing the control mechanism, and the identifier of the insulin pump remains unchanged, and thus there is no need for updating the pairing update of the identifier of the insulin pump with the personal information of the patient via the smartphone or in uploading it to a remote server, and thus the operational steps can be reduced and the patient experience can be improved.

After the CGM and/or insulin pump has been worn on the patient and started working, the CGM sends a wireless signal after completing each blood glucose monitoring, and insulin pump sends a wireless signal after completing each insulin infusion, and preferably, the wireless signal sent by the CGM and/or insulin pump is a Bluetooth signal, as previously described. Theoretically, each receiver of the receiver group 12 can receive the Bluetooth signal sent by the CGM and/or insulin pump, but after the Bluetooth signal sent by the CGM and/or insulin pump is received by the receiver that is within the optimal communication distance, the medical device stops transmitting the blood glucose data and/or insulin infusion data signal for this blood glucose monitoring circle and/or insulin infusion, which on the one hand, saves the power consumption of the medical device, and on the other hand, it can prevent that each receiver receives the data and uploads the data to the central server, which results in the duplication of the blood glucose data information and/or insulin infusion information, and occupies the storage space of the central server.

Here, the receiver that is within the optimal communication distance may mean the receiver that is the first to receive the Bluetooth signals sent by the medical device worn by the patient, and when the signaling medium is the same, e.g., when there is no obstruction or interference between all the receivers and the medical device, e.g., a wall, a metal structure, a WiFi router, a wireless phone, etc., or when there is the same obstruction and/or interference, the receiver that is within the optimal communication distance is the one that is closest to the medical device; however, when there are obstructions and/or interference that are not the same, the receiver that is within the optimal communication distance is not necessarily the one that is closest to the medical device, but rather the one that is able to first receive the Bluetooth signals sent by the medical device.

The Bluetooth signal contains the CGM's current blood glucose monitoring data information and/or insulin infusion information and the corresponding identifier information of the medical device. A receiver located within an optimal communication distance receives the CGM's current blood glucose data information and/or insulin infusion information and the corresponding identifier information, which is further uploaded to the central server 13. When the patient is in a different position, the current blood glucose data information and/or insulin infusion information and the corresponding identifier information sent will be received by different receivers located in the optimal communication position compared to the patient's positions, and the different receivers will upload the current blood glucose data information and/or insulin infusion information and the corresponding identifier information to the central server 13 after receiving them. That is, the central server 13 may store all of the blood glucose data information and/or insulin infusion information and corresponding identifier information received from the different receivers.

Therefore, the corresponding blood glucose data information and/or insulin infusion information and the identifier information, the corresponding patient personal information and the identifier information are stored in the storage module of the central server 13 at the same time. Generally, the corresponding blood glucose data information and/or insulin infusion information and the identifier information, the corresponding patient personal information and the identifier information are stored in different storage modules of the central server 13, but the two sets of data are related to each other, and the pairing of the patient personal information and the blood glucose data information and/or insulin infusion information can be accomplished by the identifier information, as shown in FIG. 5.

As mentioned earlier, when a patient is admitted to the hospital, the healthcare provider will create a new patient account through the mobile device 141 to realize the function of patient management, in addition to account management, device management, blood glucose data information and/or insulin infusion information reviewing and report printing, and so on, specifically:
In patient management, after clicking Add Account, you can scan the patient's bracelet or manually enter the patient's personal information, and at the same time scan or manually enter the identifier information of the medical device, and through the mobile phone authentication code to verify the patient's personal information and add the patient account, and when it is necessary to delete the patient account, clicking Delete to delete the corresponding account.

In account management, the mobile device 141 is set up with one or more processors that enable multiple individuals with different access level rights to modify the setup parameters of the mobile device. The supervising physician can create an HCP master account, and the HCP master account can create HCP sub-accounts to add and review all information about all patients, review the operation records of the HCP master account and the HCP sub-accounts, and the like. And the supervising physician with the HCP master account can manage hospital-wide HCP sub-accounts, departments and patients information, with read/write privileges to HCP sub-accounts, departments, and also assigns access privileges to HCP sub-accounts, e.g., a resident doctor with a HCP sub-account is assigned with editing privileges to HCP sub-accounts to modify the patient information in the department, add a patient information, and review all the patients' information, while a nurse with a HCP sub-account is assigned with privileges to HCP sub-accounts to review all the patient information in the department only.

In device management, mobile device 141 can review the status of all connected transmitters and receivers, and can add or remove transmitters and receivers, can review the status of all connected infusion mechanism and control mechanism, and can add or infusion mechanism and control mechanism.

Generally, because CGMs and insulin pumps are developed by different manufacturers, each manufacturer has a dedicated handheld device, or a dedicated APP on a mobile device to control the corresponding CGM or insulin pump, respectively, and there is no compatibility between the handheld devices or APPs of the different manufacturers, so that healthcare providers need to switch between different handheld devices or APPs when they are used at the same time, and they are also not able to review the user's glucose and insulin infusion information at the same time, which is an inconvenience for the healthcare providers to use.

In the embodiment of the present invention, the CGM and the insulin pump are developed and produced by the same manufacturer, and the compatibility of the control of the CGM and the insulin pump is taken into account during the development process, so that they can be controlled by a unified handheld device or by the same dedicated APP in the same mobile device, so that healthcare provider do not need to switch between different handheld devices or APPs when using them simultaneously, and they also can simultaneously check the user's blood glucose and insulin infusion information, and simultaneously realize the control of the CGM and/or the insulin pump, which greatly facilitates the use of the healthcare provider, and improves the healthcare provider's experience of using.

FIG. 6 is a default home screen of the APP of the mobile phone according to an embodiment of the present invention.

In this embodiment of the present invention, since the CGM and insulin infusion can be controlled by the healthcare provider through the mobile device 141, the blood glucose data information and status information of the CGM as well as the insulin infusion information and status information of the insulin pump can be displayed on the main screen of the mobile device 141. As shown in FIG. 6, the main screen includes a status bar icon area 601, and the status bar icon area 601 includes a battery icon 6011 for displaying the remaining battery status of the mobile device 141 or the battery status when charging; a time icon 6012 for displaying the current time; an audio icon 6013 for indicating different reminders, such as audio, vibration, or audio + vibration; a communication icon 6014, communicating with the insulin pump, for displaying communication connection the status with the insulin pump; alert icon 6015, for displaying alerts of different priority levels.

The main screen also displays insulin information, specifically including the insulin delivery status icon 602, insulin on board, IOB icon 603, and the remaining insulin amount icon 604 in the insulin pump, wherein the insulin delivery status icon 602 includes a graphic 6021 and/or text 6022, wherein the graphic 6021 is a ring of different colors, indicating different infusion types, and expressing the progress of infusion with a change in color, and text 6022 showing the abbreviation of the insulin infusion type and the infusion rate or progress of infusion. When the ring 6021 is gray, it indicates that the infusion device 102 is not activated; when the ring 6021 is green, it indicates that the basal infusion is in progress; when the ring 6021 is a green ring with dark green, it indicates that the temp basal infusion is in progress and the dark green part indicates the progress of the temp basal infusion; when the ring 6021 is a blue ring with dark blue, the blue ring indicates a normal bolus infusion, and the dark blue portion indicates infusion progress; when the ring 6021 is a purple ring with dark purple, the purple ring indicates an extended bolus infusion, and the dark purple portion indicates infusion progress; when the ring 6021 is red, it indicates infusion suspension. Text section 2222 Basal(U/H) 1.00 indicates the current basal infusion rate is 1.00 U/H; Temp Basal(U/H) 1.00: indicates temp basal infusion activated and temp basal rate is 1.00 U/H; Temp Basal(U/H) 1.00 85%: indicates temp basal activated, temp basal rate is 1.00 U/H and 85% of current basal rate; Normal(U) 1.00/2.00 : indicates that the normal bolus activated, 1U normal bolus infusion delivered, and the total amount of normal bolus is 2U; Extended(U) 1.00/2.00: indicates that the extended bolus activated, 1U of extended bolus infusion delivered, and the total amount of extended bolus is 2U; C-Normal(U) 1.00/2.00: normal bolus of combo bolus activated, and 1U normal bolus infusion delivered, the total amount of normal bolus in the combo bolus is 2U; C-Ext.(U) 1.00/2.00: the extended bolus of combo bolus activated, and 1U extended bolus infusion delivered, the total amount of normal bolus in the combo bolus is 2U; Suspend time remaining 0:15 indicates that the suspension has been activated and basal will resume automatically after 15 minutes. In the embodiment of the present invention, the graph 6021 and the text 6022 cooperate to jointly display the current insulin infusion status, and the user can intuitively know the current insulin infusion type, infusion rate or infusion progress and confirm the drug infusion status through both the graph and the text. In other embodiments of the present invention, the main screen can also display the current insulin infusion status only through graphics 6021 or text 6022 alone, and the user can also intuitively know the current insulin infusion type, infusion rate or infusion progress.

The main screen also displays blood glucose information, and the blood glucose information includes the blood glucose graphic display area 605 and the CGM status display area 606, when the blood glucose value is in the normal blood glucose value range, the blood glucose reading is shown in green, when the blood glucose value is lower than the low blood glucose standard value, the blood glucose reading is shown in yellow; when the blood glucose value is higher than the high blood glucose standard value, the blood glucose reading is shown in red. The blood glucose information also includes the blood glucose trend display area 607. The blood glucose trend display area 607 is indicated by indicator arrows with certain color, direction and number, to show the change trend and speed of the blood glucose reading with different color, direction and number, such as a green arrow to the right indicates that the blood glucose status is stable and tells the user that no special attention to the blood glucose is needed at present; an orange arrow to the upper right indicates that the blood glucose is slowly rising; an orange arrow to the lower right indicates that blood glucose is slowly falling, reminding the user to pay attention to further changes in blood glucose; an upward red arrow indicates that blood glucose is rising; a downward red arrow indicates that blood glucose is falling; two upward red arrows indicate that blood glucose is rising rapidly, reminding the user that he or she needs to pay attention to changes in blood glucose at all times or to start adjusting the insulin infusion strategy; two downward red cut-offs indicate a rapid drop in blood glucose, reminding the user that immediate action is needed to change the current blood glucose status, such as carbohydrate supplementation, bolus infusion, etc. Both blood glucose reading 606 and blood glucose trend indication arrow 607 are set inside the ring 6021, indicating the type and progress of insulin infusion, and the user can intuitively know the current insulin infusion status and blood glucose value and blood glucose trend at the same time.

The blood glucose graphical display area 605 includes the blood glucose reading curve or blood glucose reading discrete points 6051 over a period of time, and the normal blood glucose value area 6052, which is convenient for the user to intuitively know the blood glucose level in the past and current time period. The normal blood glucose value area 6052 is an area filled with certain colors; in another embodiment of the present invention, it is also possible to display the normal blood glucose value area only by highlighting and/or bolded the standard line of high and low blood glucose value, the user can also intuitively know the blood glucose level in the past and current time period as well as the rough blood glucose change rate.

The main screen also includes the calibration display area 608, the calibration display area 608 is a teardrop-shaped icon and indicates how long it will take for the next calibration to be completed with a different amount of teardrop color filling. Calibration of the CGM can be accomplished through the mobile device 141.

The main screen may also include a suspend infusion function icon 609, when the hypoglycemic suspend infusion function or the predictive hypoglycemic suspend infusion function in the insulin pump is turned on, i.e., when a sensor reading of the CGM triggers the suspend e function, the insulin pump performs a safety detection and automatically pauses the infusion, and the suspend infusion function icon 609 is displayed on the main screen.

It should be noted that in the embodiment of the present invention, when the patient does not use the CGM and the insulin pump at the same time, the main screen of the APP of the mobile device 141 only displays the corresponding medical data information and status information corresponding to the corresponding CGM or insulin pump.

The mobile device 141 can also set blood glucose alarms, including global blood glucose alarms and individual blood glucose alarms, the global blood glucose alarms is a default blood glucose alarms for all patients, including a high blood glucose threshold, e.g., 13.3 mmol/L; and a low blood glucose threshold, e.g., 4.4 mmol/L; a predictive blood glucose alarms, such as a rise in blood glucose values to 13.3 mmol/L or even 22.2 mmol/L in the coming period of time, e.g., half an hour, one hour, or a fall in blood glucose values to 4.4 mmol/L or even 2.2 mmol/L in the coming period of time; an alarm on the rate of change of blood glucose, such as rapid rise/rapid fall of blood glucose at a rate of 0.110 mmol/L/min , or even 0.170 mmol/L/min; a hypoglycemic alarm, e.g., blood glucose below 3.1 mmol/L. The global glucose alarm means that all patients are set to the same glucose high/low threshold, and once any patient's glucose value is monitored to reach the set threshold, the mobile device 141 will active an alarm. According to different settings, the alarms can be set as one or a combination of sound alarms, vibration alarms, and interface text alarms. The sound type, volume size, duration of the sound, frequency interval of the sound alarm; the vibration type, intensity of the vibration, duration of the vibration, frequency interval of the vibration alarm; the type of the interface text alarm, the frequency of the alarm, etc. can be personalized according to the actual needs or the patient's preferences, or refer to the contents disclosed in patent PCT/ CN2022/CN119084.

In general, the glucose alarm settings for new patients are defaulted to match the global alarm settings. If a patient's glucose alarm threshold to be set can't match the global glucose alarm threshold due to his/her own constitution or at a special stage, such as pregnancy, or due to other illnesses, specific individual glucose alarms can be set in the patient management page.

When the healthcare provider needs to review the patient's blood glucose monitoring data or prints a report, he or she enters the patient's information into the mobile device 141 or the operating system of the HIS system 142, and by the identifier and the patient's correspondence, the corresponding patient's blood glucose data information can be located in the data memory, and displayed on the monitor.

When reviewing the blood glucose data information via the mobile device 141 the current blood glucose data information and the blood glucose trend of the patient can be displayed on the real-time monitoring page of the patient management, and the blood glucose data information in a recent period of time is displayed in the form of a curve, such as three hours, six hours, nine hours, twelve hours, twenty-four hours, or forty-eight hours, and so on, and the glucose monitoring trend graph of the corresponding period of time is displayed by switching the option of different periods of time. Similarly, when you need to print a report, you can select the desired report time and report type on the report print page of patient management, and then print the report, and the electronic copy of the blood glucose report is automatically saved to the mobile device 141. The types of reports include a comprehensive report on blood glucose monitoring, an ambulatory glucose (AGP) chart, and a daily detail chart. The comprehensive report includes daily statistics and overall statistics of glucose values measured during the monitoring cycle. Statistical parameters include: number of points measured; mean value; standard deviation; coefficient of variation; average fluctuation; maximum/minimum blood glucose value; distribution of blood glucose, etc. AGP Chart displays the blood glucose AGP profile and blood glucose distribution histogram during the monitoring cycle. The following statistics are provided: CGM usage time proportion, mean value, standard deviation, coefficient of variation, estimated glycated hemoglobin, average fluctuation, average day-to-day variation, and number of high/low glucose events per day. The daily detail chart displays a graph of glycemic trends and simple statistics for each day of the monitoring cycle.

HIS system is a digital and networked management system for the various operations and medical activities of each department of the hospital, which involves all information management, including administrative management system, medical management system, decision support system, and various other auxiliary systems. The medical management system is mainly involved in the hospital's medical business information processing, mainly including outpatient, emergency management system, case management system, medical statistics query system, blood bank management system and so on. In the medical management system, you can search, delete, review and print all kinds of blood glucose data information reports of patients.

The monitoring screen 143 used at the nurses' station displays glucose data information and/or insulin infusion information for all monitored patients, and also highlights information about patients with abnormal glucose data and/or insulin infusion information, such as bed number, name, and abnormal glucose data information. Abnormal blood glucose readings are highlighted in different colors, e.g., high blood glucose is displayed in yellow, low blood glucose in red, and the patient's abnormal blood glucose data and/or insulin infusion information status is displayed individually in the form of text on the monitoring screen 143. Through the monitoring screen 143, healthcare providers, such as nurses, can quickly and clearly understand the status of all the patient's blood glucose monitoring and/or insulin infusion information, and the existence of any accidental or unexpected situation can be dealt with or feedback to the doctor in time.

The mobile device 141, the HIS system 142, and the monitoring screen 143 are different display devices that may provide different user interfaces, and the content of the interface displays, such as the amount, format and/or type of data to be displayed, alarms, and the like, may be customized by the manufacturer and/or the end user for each particular display device.

In another embodiment of the present invention, a personal diabetes management device may also be configured for a patient when the patient is in a hospital, i.e., the diabetes glucose management device used in the hospital may also include a personal diabetes management device, wherein the personal diabetes management device is matched with the medical device by the identifier information of the medical device used by the patient and communicates wirelessly. Wireless communication can be via, for example, but not limited to, radio frequency (RF) communication (e.g., radio frequency identification (RFID), Zigbee communication protocols, WiFi, infrared, wireless Universal Serial Bus (USB), Ultra Wide Band (UWB), Bluetooth^{®} communication protocols, and cellular communication, such as Code Division Multiple Access (CDMA) or Global System for Mobile Communications (GSM). Preferably, the personal diabetes management device communicates with the medical device via Bluetooth, facilitating the patient to know his/her blood glucose information and/or insulin infusion information in the first instance, and at the same time to learn and become proficient in the use of the personal diabetes management device, facilitating the use of the management device by himself/herself to monitor and manage his/her blood glucose level and infuse insulin after discharged from the hospital. When the patient is an elderly person or a young child, or a diabetic patient with a special disease who is unable to conduct self-monitoring of blood glucose and insulin infusion, healthcare providers can limit the patient's operation on the personal diabetes management device through the lock mode on the mobile device 141, so that the patient is unable to review the real-time glucose data information and/or insulin infusion information, receive glucose alarms, or change the alarm settings on the personal diabetes management device, etc.. Therefore, it can prevent the misuse of the personal diabetes management device by the patient, such as deleting the device, releasing the connection relationship between the personal diabetes management device and the medical device, which may affect the normal glucose monitoring and/or insulin infusion; not accepting the blood glucose alarm also prevents the blood glucose alarm from interfering with or affecting the patient.

In embodiments of the present invention, the personal diabetes management device may also control both the CGM and/or the insulin pump.

In other embodiments of the present invention, a diabetes management device for use within a hospital may further comprise a secondary management device, the secondary management device also being matched to the medical device via identifier information of the medical device used by the patient, and communicating wirelessly. Wireless communication can be via, for example, but not limited to, radio frequency (RF) communication (e.g., radio frequency identification (RFID), Zigbee communication protocols, WiFi, infrared, wireless Universal Serial Bus (USB), Ultra Wide Band (UWB), Bluetooth^{®} communication protocols, and cellular communication, such as Code Division Multiple Access (CDMA) or Global System for Mobile Communications (GSM). Preferably, the secondary management device communicates with the medical device via Bluetooth. Although the patient can perform blood glucose monitoring and/or insulin infusion by himself or herself, the guardian or healthcare provider may also use the secondary management device in order to keep track of the patient's blood glucose level and/or insulin infusion state, at that time the content displayed by the secondary management device is the same as the content displayed by the personal diabetes management device. When the user of the secondary management device is a healthcare provider, the secondary management device and the mobile device 141 of the healthcare provider's end-user device can be the same device.

In summary, the present invention discloses a diabetes management system for use in a hospital. The system comprises at least a wearable medical device, a plurality of receivers, a central server and a healthcare provider terminal, the receivers receive the data information sent by the medical device and upload it to the central server, and the healthcare provider communicates with the central server and displays or reviews the data information through the device, as any one of the multiple receivers can receive the data information sent by the medical device and upload it to the central server, as a result, the patient's range of motion in the hospital is no longer restricted, which improves the patient's experience and keeps the patient in a comfortable mood, while the healthcare provider is able to know the patient's blood glucose level and/or insulin infusion state in real time through the device used by the healthcare provider and deal with the abnormalities in a timely manner, so that the patient's blood glucose level can be maintained at a stable level.

While the invention has been described in detail with reference to the specific embodiments of the present invention, it should be understood that it will be appreciated by those skilled in the art that the above embodiments may be modified without departing from the scope and spirit of the invention. The scope of the invention is defined by the appended claims.

## Claims

1. A diabetes management system for use in a hospital, comprising:
at least one wearable medical device worn by at least a patient, wherein the wearable medical device comprises at least a memory, a processor and a communication interface, and the communication interface is configured to transmit and receive information;
a plurality of receivers, any of which is configured to receive and relay information transmitted by the wearable medical device;
a central server, comprising a memory and a communication interface configured to communicate with an external device and store information received from the external device; and
a healthcare provider terminal, configured to communicate with the central server and to display or review medical data information.

2. The diabetes management system for use in a hospital of claim 1, wherein,
the at least one wearable medical device comprises a CGM and/or an insulin pump.

3. The diabetes management system for use in a hospital of claim 2, wherein,
when a position of the at least one wearable medical device changes with the patient in the hospital, the information transmitted by the wearable medical device is received by the receiver located at an optimal communication distance.

4. The diabetes management system for use in a hospital of claim 3, wherein,
each wearable medical device is provided with a unique identifier and the information transmitted by the wearable medical device at least comprises the identifier information.

5. The diabetes management system for use in a hospital of claim 4, wherein,
the healthcare provider binds and uploads the patient's personal information and the identifier information to the central server via the healthcare provider terminal.

6. The diabetes management system for use in a hospital of claim 5, wherein,
the identifier includes at least one of a device identifier, a hardware identifier, a generic unique identifier, a serial number, a communication protocol-based identifier, a manufacturer's identifier.

7. The diabetes management system for use in a hospital of claim 6, wherein,
the identifier is provided on an outer packaging or housing of the wearable medical device.

8. The diabetes management system for use in a hospital of claim 7, wherein,
the wearable medical device at least comprises a reusable part, and the identifier is provided on the outer packaging or housing of the reusable part.

9. The diabetes management system for use in a hospital of claim 8, wherein,
the identifier is set in the form of QR codes, bar codes, or NFC tags.

10. The diabetes management system for use in a hospital of claim 2, wherein,
the healthcare provider terminal comprises one or more processors that enable multiple individuals with different access level rights to modify the setup parameters of the healthcare provider terminal.

11. Th diabetes management system for use in a hospital of claim 10, wherein,
the healthcare provider terminal is a mobile device and the CGM and/or the insulin pump is controlled by the same APP in the mobile device.

12. The diabetes management system for use in a hospital of claim 1, wherein,
the healthcare provider terminal further comprises a displayer, at least used for displaying one of the patient's real-time glucose data, historical glucose trend graph or insulin infusion information.

13. The diabetes management system for use in a hospital of claim 1, wherein,
the healthcare provider terminal is a HIS system, configured to review, print or delete blood glucose and/or insulin infusion reports.

14. The diabetes management system for use in a hospital of claim 1, wherein,
the healthcare provider terminal is a monitoring screen, configured to display blood glucose and/or insulin infusion information for all patients being monitored.

15. The diabetes management system for use in a hospital of claim 15, wherein,
the monitoring screen highlights the patient's abnormal blood glucose and/or insulin infusion information.
